# FASCICULE DE BREVET EUROPEEN

(11) **EP 4 256 327 B1**
(45) Date de publication et mention de la délivrance du brevet: **11.02.2026**
(21) Numéro de dépôt: 21835636.8
(22) Date de dépôt: 03.12.2021
(51) Int. Cl.: G01N 33/483, G01N 33/487, A61B 5/262, A61B 5/263, A61B 5/279, A61B 5/00, B82Y 5/00, B82Y 30/00

(54) **PLATEFORME DE NANOSTRUCTURES POUR L'INTERFAÇAGE CELLULAIRE ET PROCÉDÉ DE FABRICATION CORRESPONDANT**
NANOSTRUKTURPLATTFORM FÜR ZELLULARE SCHNITTSTELLE UND ENTSPRECHENDES HERSTELLUNGSVERFAHREN
NANOSTRUCTURE PLATFORM FOR CELLULAR INTERFACING AND CORRESPONDING PRODUCTION METHOD

(30) Priorité: 04.12.2020 FR 2012720; 22.10.2021 FR 2111269
(43) Date de publication de la demande: 11.10.2023
(73) Titulaire: Centre National de la Recherche Scientifique (CNRS), 75016 Paris Cedex 16 (FR)
(72) Inventeur: LARRIEU, Guilhem, 31290 Cessales (FR)
(74) Mandataire: Vidon Brevets & Stratégie
(86) Numéro de dépôt international: PCT/EP2021/084283
(87) Numéro de publication internationale: WO 2022/117880

(56) Documents cités:
- WO-A1-2020/035570
- WO-A2-2019/217553
- US-A1- 2007 187 840
- US-A1- 2018 169 403
- CASANOVA A ET AL: "Nanowire based bioprobes for electrical monitoring of electrogenic cells", JOURNAL OF PHYSICS: CONDENSED MATTER, INSTITUTE OF PHYSICS PUBLISHING, BRISTOL, GB, vol. 30, no. 46, 29 October 2018 (2018-10-29), pages 464001, XP020332027, ISSN: 0953-8984, [retrieved on 20181029], DOI: 10.1088/1361-648X/AAE5AA
- JACOB T. ROBINSON ET AL: "Vertical nanowire electrode arrays as a scalable platform for intracellular interfacing to neuronal circuits", NATURE NANOTECHNOLOGY, vol. 7, no. 3, 10 January 2012 (2012-01-10), London, pages 180 - 184, XP055248720, ISSN: 1748-3387, DOI: 10.1038/nnano.2011.249
- LIU REN ET AL: "High Density Individually Addressable Nanowire Arrays Record Intracellular Activity from Primary Rodent and Human Stem Cell Derived Neurons", NANO LETTERS, vol. 17, no. 5, 10 May 2017 (2017-05-10), US, pages 2757 - 2764, XP055828877, ISSN: 1530-6984, DOI: 10.1021/acs.nanolett.6b04752
- LEE SEULAH ET AL: "Formation of Vertically Aligned Cobalt Silicide Nanowire Arrays Through a Solid-State Reaction", IEEE TRANSACTIONS ON NANOTECHNOLOGY, IEEE SERVICE CENTER, PISCATAWAY, NJ, US, vol. 12, no. 5, 18 June 2013 (2013-06-18), pages 704 - 711, XP011525833, ISSN: 1536-125X, [retrieved on 20130904], DOI: 10.1109/TNANO.2013.2268578

## Description

### 1. Domaine

La présente technique se rapporte aux domaines des nanostructures et plus particulièrement des nanostructures à destination de captation de phénomènes biologiques. Plus particulièrement, la présente technique se rapporte à une plateforme de mesure et de stimulation de cellules de type neurales ou encore de cardiomyocytes, et plus généralement à tout type de cellules dont il est envisageable de capter une activité électrique ou électrophysiologique, pouvant être des cultures cellulaires dissociées (neurones, cellules musculaires, cellules cardiaques ...), des cultures tissulaires d'organe ou des tranches de tissus (hippocampe, cervelet, moelle épinière, rétine, ...) ou des organoïdes à base de cellules dérivées de cellule souches. La présente technique peut également être mise en œuvre in vivo, pour l'interfaçage avec des cellules au niveau d'organes fonctionnels.

### 2. Art Antérieur

L'étude des états électrophysiologiques des cellules excitables (neurones, cardiomyocytes) et des groupes et réseaux formés par ces cellules permet d'améliorer la compréhension des états fonctionnels et pathologiques des organes auxquels ces cellules se rapportent. L'état électrophysiologique de la cellule est traditionnellement étudié avec de l'imagerie calcique, qui nécessite l'utilisation de composant chimique pouvant perturber le développement et l'activité cellulaires ainsi que des dispositifs miniaturisés permettant la mesure directe des potentiels (ou courant) électriques au niveau cellulaire. Ces sondes qui offrent une affinité spécifique avec les cellules, i.e. membranes externes voir dans certains cas un accès à l'intérieur (cytoplasme) de la cellule, permettant ainsi les mesures des variations de potentiels d'une ou plusieurs cellules dans un réseau de cellules. Ces sondes comprennent des « patch-clamp » avec micropipette en verre, des matrices de microélectrodes/ nanoélectrodes (MEAs/NEAs), et des transistors à effet de champs (FET) planaires ou à base de nanofils, et avec différents matériaux, tels que des réseaux de nanofils verticaux en silicium (Si), platine (Pt), oxyde d'iridium (IrOx) etc.

Des électrodes de micropipette en verre avec un procédé de « patch-clamp » ont traditionnellement été utilisées. Elles permettent de réaliser des mesures de bonne qualité, mais d'une part elles ne peuvent pas être utilisées pour un grand nombre de cellules de manière simultanée (de fait de la difficulté de mise en œuvre de ces sondes) et d'autre part elles entrainent la mort des cellules à relativement court terme, de fait de la pénétration de la micropipette au sein de la cellule.

Plus récemment, les développements technologiques ont permis de développer des réseaux de microélectrodes planaires qui sont en passe de devenir des plateformes standards de l'étude des réponses électrophysiologiques des réseaux cellulaires sur de longues périodes (plusieurs semaines). Ces réseaux de microélectrodes présentent l'avantage de ne pas altérer l'enveloppe des cellules et donc de ne pas entrainer la mort prématurée de celles-ci. Cependant, les réseaux de microélectrodes planaires présentent l'inconvénient d'être d'une taille relativement grande comparée à celle d'une cellule (donc captent l'information provenant de multiples cellules en même temps). Plus important encore la mesure des variations de potentiels est une donnée importante qu'il convient de mesurer. Le problème principal réside cependant de ces réseaux existants et de ces plateformes est la faible interaction cellule/microélectrodes qui induit des signaux (potentiel d'action) très dégradés en termes d'amplitude et donc difficilement interprétables.

Les plateformes et réseaux à base de microélectrodes ont fait l'objet de développements technologiques importants, tels que ceux présentés dans les documents US7,905,013, WO2017127551 et WO2019110485.

Dans US7,905,013, une couche diélectrique sur une couche conductrice est gravée sélectivement à l'état humide, formant des trous de contact avec des parois inclinées dans la couche diélectrique et exposant des régions de la couche conductrice. Des interfaces neuronales de nanofils IrOx sont ensuite développées à partir des régions exposées de la couche conductrice. Les interfaces neuronales à nanofils IrOx ont chacune une section transversale comprise entre 0,5 et 10 micromètres, une hauteur moyenne comprise entre environ 10 nanomètres (nm) et environ 10 micromètres (µm) et un diamètre d'extrémité proximal moyen dans une plage d'environ 1 nm à environ 1 µm. Zhang rapporte des grappes de sondes sur des puces allant de 1 à 100 millimètres carrés. Les groupes comprennent chacun de 2 à jusqu'à 12 électrodes, situées dans un diamètre de groupe compris entre 5 et 50 micromètres, le nombre de groupes sur la puce étant compris entre 2 et 100.

Dans WO2017127551, un réseau de capteurs à sonde neurale est décrit sur un substrat isolant. Un tel réseau comprenant un substrat avec un motif métallique sur celui-ci. Un réseau de sondes de nanofils verticaux semi-conducteurs s'étend à distance du substrat, ont été structurées dans une couche active de semiconducteur déposée sur ce substrat isolant. Les sondes sont adressées électriquement, de manière individuelle, à travers le motif métallique. Le motif métallique est isolé avec un diélectrique, et les parties de base et les tiges des nanofils sont également de préférence isolées. Cette plateforme permet de stimuler individuellement les cellules qui prennent place sur les nanofils.

Dans WO2019110485, une plateforme comprenant à la fois des nanofet et des nanofils est décrite. Cette plateforme est fabriquée par voie descendante. Plus particulièrement, le procédé de fabrication d'une telle plateforme suppose l'utilisation d'une base comprenant un substrat sur lequel est déposée une couche de 1µm de dioxyde de silicium (SiO2), elle-même recouverte d'une couche de silicium (Si) monocristallin de 4-5µm d'épaisseur. C'est cette dernière couche de silicium mono cristallin qui fait l'objet d'un traitement permettant à la fois de créer les nanofil et les nanofet. La technique proposée dans WO2019110485 est efficace en ce qu'elle permet de répondre aux problématiques posées notamment par les techniques de l'art antérieur (US7,905,013 et WO2017127551). En revanche, la technique de WO2019110485 souffre d'un problème d'homogénéité lié à la couche de silicium (Si) monocristallin. En effet les inventeurs ont constaté, en conditions opérationnelles, que la couche active peut connaitre, dans certaines circonstances, une variation d'épaisseur de plus ou moins 500 nm, soit environ dix pour cent de l'épaisseur théorique globale de la couche active. Une telle variation impacte fortement les rendements de fabrication des plateformes avec la technique proposée dans WO2019110485. De plus, cette approche n'est possible qu'en utilisant un substrat très spécifique (silicium sur isolant), avec une couche active d'épaisseur fixée.

Pour ce qui est de l'implémentation sur CMOS, le document US20180169403A1 expose la technique communément mise en œuvre. Dans ce document l'approche est la structuration directe de chaque pixel par l'intermédiaire d'une couche, ajoutée par-dessus le CMOS, supposément d'épaisseur finie, qui est ensuite structurée, déterminant la longueur finale des nanostructures. En plus des problèmes précités, cela aussi introduit des contraintes sur les procédés utilisés devant être compatible avec le pixel métallique menant au CMOS. Cela peut aussi introduire des problèmes de fiabilité sur le circuit CMOS car les connexions métalliques y menant sont non protégées (puisqu'elles sont structurées en tant que telle).

Toutes ces approches souffrent aussi de l'inhomogénéité de vitesse de gravure sèche pour créer les nanostructures : à l'échelle d'un substrat la vitesse de gravure est toujours plus rapide sur les zones périphériques que sur le centre introduisant des centaines de nanomètres de « sur gravure » au bord par rapport au centre. Ainsi sur une couche d'épaisseur définie, il n'est pas possible d'atteindre la hauteur visée sur tous les endroits du substrat.

En résumé, l'approche suivie par l'homme du métier consiste à structurer les nanoélectrodes verticales dans une couche d'épaisseur définie, cette épaisseur induisant la longueur des nanostructures. Cette approche, comme présentée précédemment, fait face à de nombreux problèmes comme l'introduction d'une couche active uniforme (souvent associée à l'utilisation de substrat très spécifique et couteux), la structuration de la couche de manière homogène, la forte interaction entre la création de la couche d'interface sur les nanostructures et les accès métalliques de celle-ci (ou du pixels CMOS)

Il est donc nécessaire de proposer une technique de fabrication pouvant être implémenté sur une variété de substrat qui permette l'obtention de plateformes qui assurent de pouvoir effectuer des mesures et des observations de manière fiable, tout en garantissant la survie à moyen ou long terme des cultures de cellules et en garantissant une reproductibilité de fabrication assurant un haut rendement, une parfaite compatibilité avec les techniques classiques de micro-technologie disponible en fonderie, tout en simplifiant le procédé et donc entrainant une baisse des couts de fabrication.

Une plateforme pour interfaçage cellulaire est aussi connue de CASANOVA A ET AL: "Nanowire based bioprobes for electrical monitoring of electrogenic cells", JOURNAL OF PHYSICS: CONDENSED MATTER, INSTITUTE OF PHYSICS PUBLISHING, BRISTOL, GB, vol. 30, no. 46, 29 octobre 2018 (2018-10-29), page 464001, XP020332027,ISSN: 0953-8984, DOI: 10.1088/1361-648X/AAE5AA.

### 3. Résumé

La présente technique a été construite sur la base de ces problèmes de l'art antérieur. Plus particulièrement, la présente technique se rapporte à un procédé de fabrication d'une plateforme pour l'interfaçage cellulaire, ladite plateforme comprenant au mains une nanosonde et ladite plateforme étant fabriqué sur un substrat massif prédéterminé, dépourvu de couche active. La technique proposée est remarquable en ce que le procédé est de type descendant, et que sur le substrat massif prédéterminé, dépourvu de couche active il comprend les étapes ordonnées suivantes :
- création de nanofils verticaux sur le substrat massif, destines a former ladite au mains une nanosonde;
- dépôt d'une couche de diélectrique isolant, lorsque nécessaire ;
- dépôt d'une couche de silicium ;
- création des lignes d'accès aux nanofils ;
- siliciuration des lignes d'accès et des nanofils ;
- structuration métallique des lignes d'accès ;
- dépôt de couche isolant pour la mesure en milieu liquide ;
- retrait sélectif de la couche d'isolant sur les nanofils de ladite au mains une nanosonde.

Ainsi, il est possible de fournir une plateforme dont les propriétés sont stables et reproductibles. En effet, dans la mesure ou la structuration verticale du substrat a lieu avant la création des lignes d'accès (laquelle est effectuée par dépôt de silicium par exemple), il est possible de contrôler tant la hauteur des nanofils verticaux que l'épaisseur des lignes d'accès aux nanofils verticaux. Le contrôle de ces deux paramètres permet ainsi de disposer de plateforme dont les caractéristiques sont connues et constantes lors de la fabrication.

Selon une caractéristique particulière, le substrat massif prédéterminé appartient au groupe comprenant : substrat transparent (par exemple quartz, silice fondue), substrat flexible (par exemple polyimide), silicium (ou tout autre semiconducteur), une couche de passivation (SiO, SiN) d'un circuit électronique CMOS.

Ainsi, il est possible de fabriquer une plateforme adaptée à des besoins spécifiques. Notamment, il est possible de disposer d'une plateforme sur un substrat transparent tel que le quartz, en réalisant une structuration verticale directement sur le substrat de quartz, sans nécessiter l'adjonction d'une couche active de type Si à nanostructurer, comme dans l'art antérieur. Il est également possible de disposer d'une plateforme directement construite sur une électronique de type CMOS, de sorte que les signaux électriques de la plateforme soient traités, en totalité ou en partie, directement par cette plateforme sans structuration directe des pixels, comme dans l'art antérieur.

Selon une caractéristique particulière, l'étape de création des nanofils sur le substrat massif prédéterminé comprend :
- Une étape de photolithographie, comprenant un dépôt local d'un motif de résine qui sert de masque de protection ; et
- Une étape de gravure sèche par bombardement d'ions permettant l'obtention des nanofils verticaux sur le substrat, à la hauteur souhaitée.

Ainsi, il est possible de définir facilement quels sont les différents schémas de structuration des nanofils verticaux sur la plateforme, tout en contrôlant efficacement la hauteur finale de ces nanofils. Lorsque le substrat est électriquement conducteur (Si par exemple), l'étape de nanostructuration est suivie d'un dépôt pleine plaque d'une couche d'isolant (SiO2 ...) d'une épaisseur comprise entre 20nm à 200 nm, épaisseur permettant de remplir les objectifs de dépôt ultérieurs des autres composés, tout en garantissant l'utilisabilité des plateformes vis-à-vis des matériels biologiques étudiés ou stimulés.

Par ailleurs, il peut y avoir une couche intermédiaire (appelé masque dur) lors de l'étape de gravure. Cette couche est structurée par une première gravure des motifs en résine (définissant ainsi un masque pour une deuxième gravure des nanofils). Ceci permet l'utilisation de motifs qui résiste mieux à la gravure que la résine et ainsi obtenir des nanofils plus longs plus facilement.

Selon une caractéristique particulière, l'étape de création des lignes d'accès aux nanofils comprend :
- Une étape de dépôt d'une couche de polysilicium, pleine plaque, par CVD ;
- Une étape de photolithographie, comprenant un dépôt local d'un motif de résine qui sert de masque de protection sur les lignes d'accès et les nanofils verticaux ; et
- Une étape de gravure sèche par bombardement d'ions pour retrait de la couche de polysilicium aux emplacements non désirés.

Selon un mode de réalisation particulier, la couche de polysilicium a une épaisseur comprise entre 20 et 200 nm.

Selon un mode de réalisation particulier, la couche de polysilicium a une épaisseur d'environ 100 nm.

Ainsi, cette gamme d'épaisseur permet de ne pas rendre les nanostructures trop large tout en permettant de minimiser les résistances d'accès. Selon un autre mode de réalisation, la polysilicium peut également être remplacé par du silicium amorphe. D'une manière générale il peut s'agir de silicium.

Selon un autre aspect, la présente divulgation se rapporte à une plateforme selon la revendication 9.

Selon un autre aspect, la présente divulgation se rapporte à un microprocesseur comprenant une plateforme selon la revendication 9.

Cet objet assure un bon interfaçage avec les structures cellulaires tout en assurant une captation de signal efficace en provenance des cellules interfaçées.

Selon une caractéristique particulière, une ligne d'accès à un nanofil permet de relier le nanofil directement à un transistor du microprocesseur par l'intermédiaire d'un via.

### 4. Figures

D'autres caractéristiques et avantages apparaîtront plus clairement à la lecture de la description suivante d'un mode de réalisation préférentiel, donné à titre de simple exemple illustratif et non limitatif, et des dessins annexés, parmi lesquels :
- [fig 1] décrit le principe les étapes mises en œuvre pour la fabrication d'une plateforme selon la présente technique ;
- [fig 2] représente des nanofils verticaux obtenus sur un substrat de silicium ;
- [fig 3] représente des nanofils verticaux obtenus sur un substrat de quartz ;
- [fig 4] représente un nanofil vertical d'une hauteur significativement supérieure à celle des nanofils de l'art antérieur ;
- [fig 5] représente une plateforme d'interfaçage comprenant sept nanofils verticaux, composées de nanofils structurés directement dans le substrat de Si massif, puis recouvert d'une couche d'oxyde (SiO2) et d'une couche de Poly-Si qui est structurée pour former une électrode comprenant les 7 nanofils prolongé par un chemin d'accès électrique ; finalement, cette couche de Si a été siliciurée sélectivement en PtSi;
- [fig 6] est une vue de coupe réalisée sur l'électrode de la Fig.5 représentant deux nanofils verticaux, laissant apparaitre l'empilement des couches ainsi réalisées selon le procédé de l'invention;
- [Fig. 7] représente des nanofils verticaux obtenus sur une couche de passivation de circuit CMOS. Les nanofils, en SiOx, sont structurés dans la couche de passivation finale du circuit et sont recouverts d'une couche de Si amorphe (^{~}90nm) ;
- [fig. 8] représente une électrode à 3 nanofils où un dépôt localisé par électrochimie d'un matériau organique conducteur (PEDOT:PSS) a été réalisé ;
- [fig 9] représente schématiquement les différentes étapes du procédé de l'invention ;
- [fig 10] représente schématiquement les différentes étapes du procédé de l'invention implémentées dans la couche de passivation d'un circuit CMOS ;
- [fig 11] est une coupe d'une sonde et d'un via pour l'atteinte d'un pixel d'un circuit CMOS telle qu'elle a été mise en œuvre par la présente technique ;
- [fig 12] représente un ensemble de nanoélectrodes sur HDCMOS telles qu'elles ont été mises en œuvre par la présente technique ;
- [fig 13] représente des nanofils sur quartz tels qu'ils ont été mis en œuvre par la présente technique ;
- [fig 14] représente des nanofils sur quartz avec PtSi tels qu'ils ont été mis en œuvre par la présente technique ;
- [fig 15] représente, dans une vue plus large, des nanofils sur quartz avec PtSi tels qu'ils ont été mis en œuvre par la présente technique ;
- [fig 16] représente, dans une vue plus rapprochée, un nanofil sur quartz avec PtSi , reportant les différentes mesures de ce nanofil, tel qu'ils a été mis en œuvre par la présente technique ;
- [fig 17] représente un nanofil sur quartz avec Si tels qu'ils ont été mis en œuvre par la présente technique.

### 5. Description détaillée

Comme explicité précédemment, les techniques antérieures souffrent de problématiques notamment liées à la répétabilité de fabrication et à l'industrialisation massive de la fabrication des plateformes, ces deux contraintes étant importantes pour offrir des produits économiquement intéressants. Plus particulièrement, la technique antérieure consistant à attaquer une surface active d'un substrat pour y former les nanosondes permet certes l'obtention de résultats satisfaisants, mais souffre de résultats aléatoires liés à l'inconstance de l'épaisseur de cette couche active que l'on vient attaquer et à des variations des vitesses d'attaques en elle-même en gravure plasma directive.

Par exemple, lorsque la couche active du substrat à graver est supposée mesurer 5µm, le procédé antérieur consiste, en premier lieu, à attaquer cette couche active, par exemple sur 4 µm, de sorte à délivrer une couche restante (par exemple pour une ligne d'accès) d'une épaisseur de 1µm et des nanosondes d'une hauteur donnée (hauteur d'au maximum l'épaisseur de la couche active à graver). Or, lorsque l'épaisseur de la couche active est différente et fluctuante sur la surface du substrat, l'épaisseur de la couche active (par exemple de la ligne d'accès) finalement obtenue n'est plus constante : elle peut varier d'un endroit à l'autre du substrat de plusieurs centaines de nanomètres. Ceci est problématique car, à cette épaisseur (centaines de nanomètres), les caractéristiques électriques de la plateforme (par exemple les caractéristiques électriques de la ligne d'accès ou celle de la nanosonde) varient en fonction de l'épaisseur. Il en découle que les mesures électriques réalisées sont plus ou moins faussées et/ou fiables en fonction de l'épaisseur finale de la couche active une fois « structurée ». Comme indiqué précédemment, pour une épaisseur théorique de 5µm (5 micromètres), la variation d'épaisseur peut être de plus ou moins 500 nanomètres. Pour reprendre l'exemple d'une ligne d'accès, l'épaisseur de celle-ci peut donc varier théoriquement de 0 à 1,5 µm, ce qui est potentiellement dommageable en termes de constance de fabrication et de reproductibilité.

Pour pallier ce problème d'origine, les inventeurs ont eu l'idée de développer un nouveau procédé de fabrication qui apporte plus de constance dans la hauteur des composants formés sur la plateforme et se libère des contraintes de la structuration d'une couche dont l'épaisseur est directement liée aux dimensions des nanostructures fabriquées. Dans ce nouveau procédé, on inverse le paradigme de fabrication de sorte à apporter une certaine reproductibilité des composants : la couche active (i.e. celle dans laquelle la nanostructure est réalisée jusqu'à l'isolant) n'est plus celle qui est utilisée pour la nanostructuration. Plus particulièrement, au lieu de vouloir structurer une couche active qui est ajoutée sur le substrat (dans le cas d'un substrat SOI ou SOQ) ou aux pixels du CMOS (dans le cas d'une intégration sur circuit électronique), comme cela est traditionnellement le cas des méthodes antérieures, l'invention consiste à structurer la partie supérieure d'une couche très épaisse (i.e. un substrat massif, de silicium, quartz, substrat flexible), puis à y déposer une couche active d'épaisseur contrôlée de l'ordre de la centaine de nanomètres selon une méthode prédéterminée. Ce dépôt d'une couche active permet d'assurer la constance de l'épaisseur de ce dépôt et donc d'assurer que les caractéristiques électriques des différents dispositifs de la plateforme sont constantes.

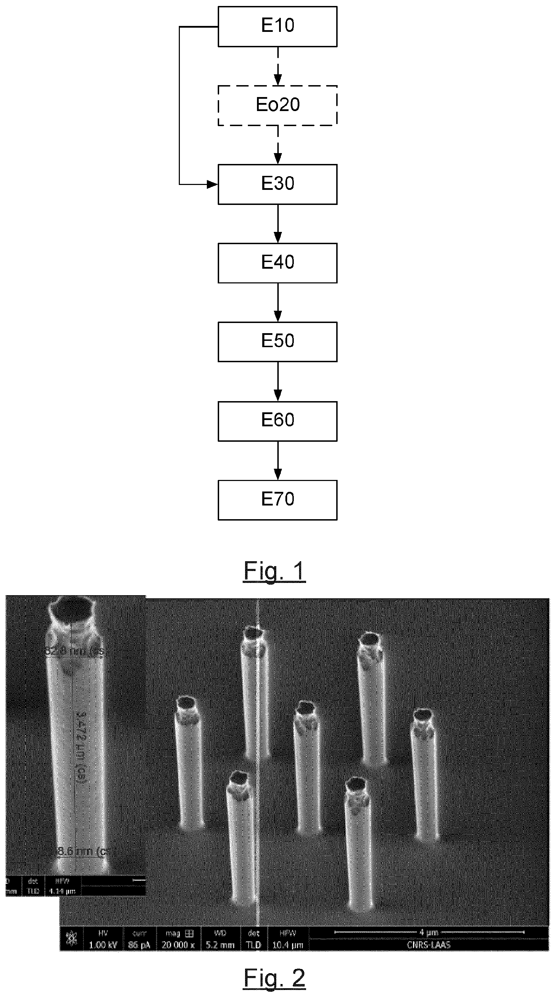

En relation avec la figure 1, d'une manière générale, la méthode de fabrication proposée comprend ainsi, postérieurement à l'obtention d'un substrat convenant à la fonction souhaitée :
- une étape de structuration (E10) du substrat, pour y créer la topologie des structures de nanosondes ;
- une étape de dépôt d'une couche de SiO2, lorsque nécessaire ;
   éventuellement, une étape de dépôt (Eo20) d'une couche isolante lorsque le substrat est conducteur (cas du silicium ou du carbone ou de tout autre semiconducteur ou métal, par exemple) ;
- une étape de dépôt (E30) d'une couche active sur ce substrat, selon un modèle adapté à la fonction recherchée pour les dispositifs de la plateforme ; l'épaisseur de la couche active déposée est contrôlée et assure la reproductibilité des plateformes obtenues ; la couche active déposée est du silicium (silicium polycristallin, aussi appelé poly-Si par LPCVD ou silicium amorphe basse température par PECVD) ;
- une étape de définition (E40) des électrodes ;
- une étape de dépôt (E50) d'une couche d'interface sonde ;

En d'autres termes, on dépose du silicium afin de pouvoir réaliser une approche maitrisée en nano électrique qui est la siliciuration sélective (tel que réalisée ci-après), on structure la couche de silicium, puis on dépose du métal partout, qui lors d'une activation thermique ne réagit (alliage Si-Métal) seulement aux endroits où il est en contact avec le silicium. Ainsi, lors d'une gravure chimique dite sélective on enlève le métal sur les zones non réagit sans attaquer l'alliage formé. L'avantage est que l'on n'a pas d'étape de lithographie à effectuer ; On pourrait à la place déposer directement un métal, puis faire un masquage de résine et graver le métal sur les zones non protégées par la résine ; Cette approche est moins rependue dans les fonderies de microélectronique Si, mais reste envisageable ;
- une étape de structuration (E60) des lignes d'accès métalliques ;
- une étape de dépôt (E60) d'une couche isolante ;
- une étape de retrait sélectif (E70) de la couche isolante (par exemple sur les nanosondes ou tout autre emplacement approprié en fonction de la plateforme considérée).

Grace à la mise en œuvre ordonnée de ces étapes, il est possible d'obtenir des sondes ayant toutes les mêmes dimensions (c'est-à-dire ayant les hauteurs souhaitées) pour la captation de phénomènes biologiques, tout en garantissant l'épaisseur des lignes d'accès (et donc garantissant une constance des caractéristiques électriques) et en assurant une forte reproductibilité (et donc un passage à l'échelle industrielle). De plus, les lignes d'accès sont plus homogènes et moins hautes : elles ne sont donc plus « guidantes » pour les cellules en culture, ce qui présente un avantage certain en termes de développement naturel de l'échantillon biologique sur la plateforme.

Par ailleurs, les techniques décrites étant basées sur un substrat « brut » (i.e. ne comprenant pas de couche active), il est possible de définir des hauteurs de sondes différentes en fonction de l'usage souhaité. Plus particulièrement, il est possible de définir des sondes de hauteurs différentes en fonction de la localisation sur une même plateforme. Il est également possible de définir des hauteurs de sondes supérieures à celles antérieures (limitées par l'épaisseur de la couche active).

La technique décrite met en œuvre une structuration des nanosondes directement sur substrat massif (Si ou quartz par ex.). Elle permet de porter cette technologie de manière très simple sur des substrats à fort intérêt, comme des substrats transparents (quartz) ou des substrats flexibles, substrats de choix pour des applications in vivo.

La technique décrite permet d'obtenir un rendement de fabrication des puces maximal : l'épaisseur des lignes d'accès correspond à l'épaisseur du silicium déposé (entre 50 et 200 nanomètres, par exemple 100 nm) par CVD où la variation d'épaisseur de moins de 2% à l'échelle du substrat entier. L'épaisseur du silicium déposé doit être relativement fine afin d'éviter de trop augmenter les diamètres des nanostructures par les épaisseurs successives déposées sur les structures.

On décrit, en relation avec les figures 2 à 8, une vue partielle d'une plateforme de nanosondes obtenues par l'intermédiaire du procédé précédemment décrit.

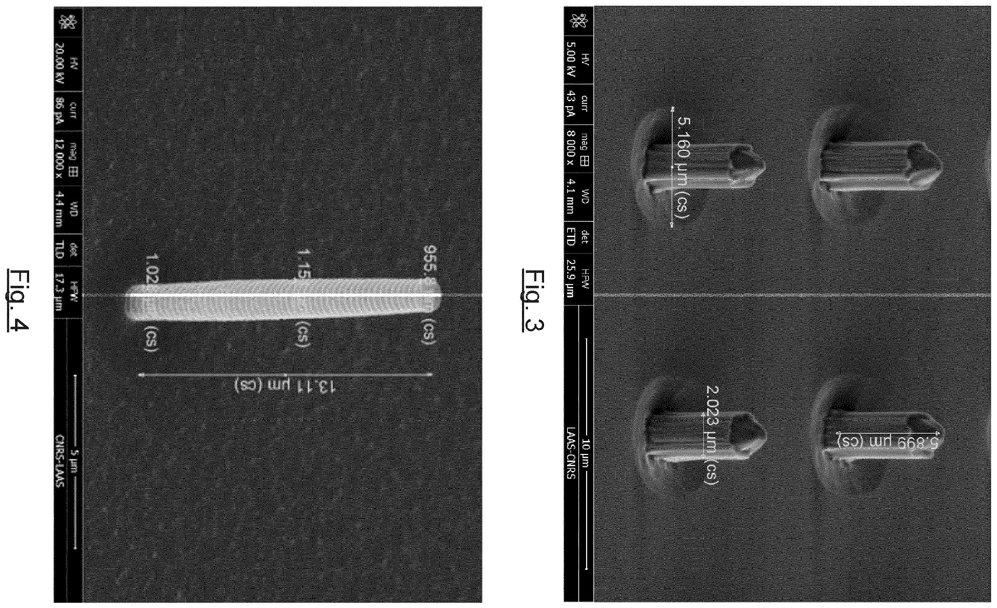

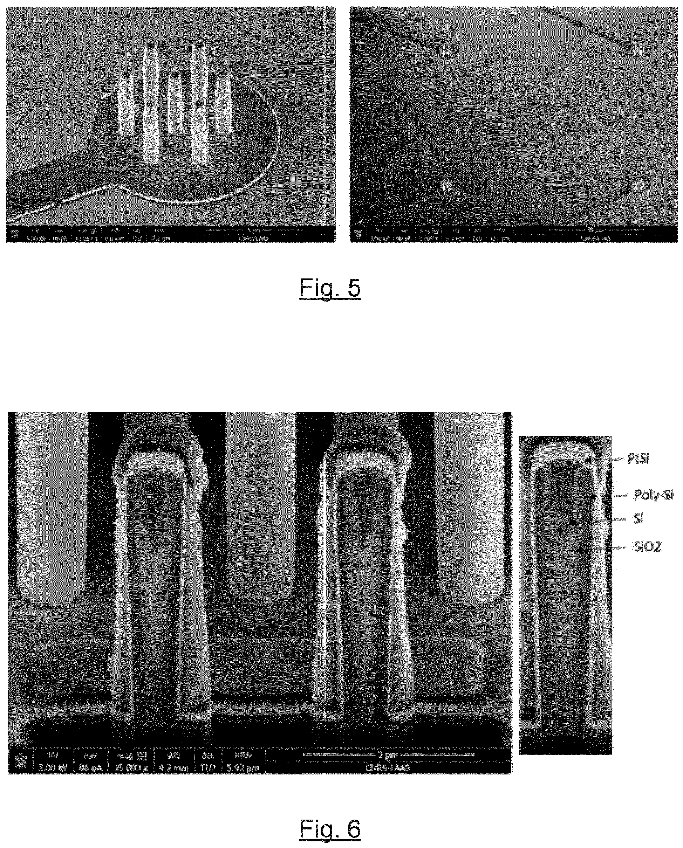

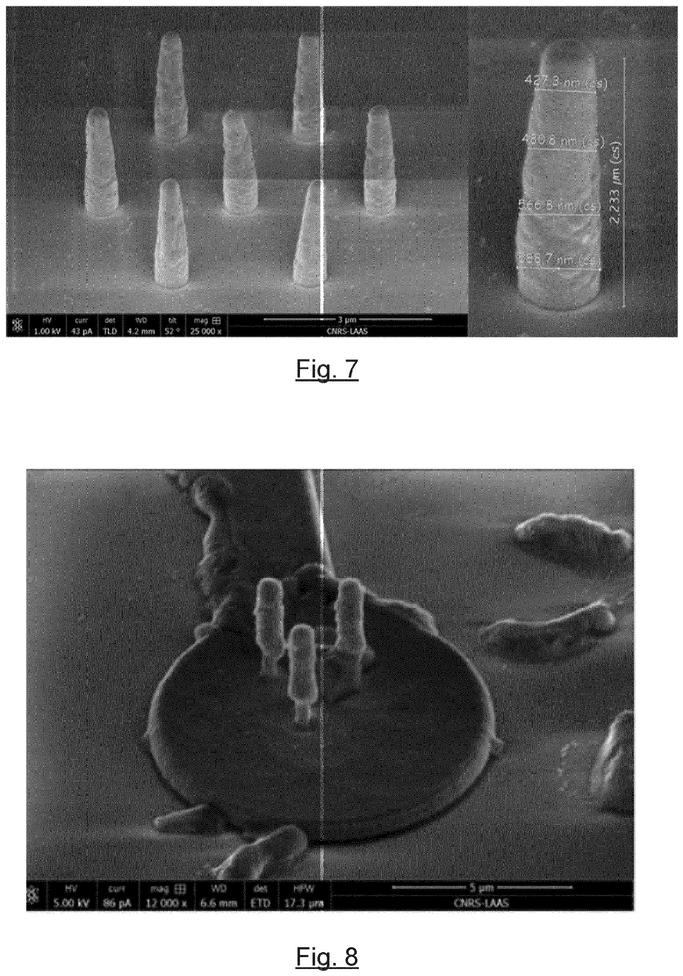

La figure 2 présente le résultat d'une structuration directe sur un substrat massif de silicium, avant mise en œuvre des étapes de traitement de surfaces, d'isolation et de structuration. Sur cette figure 2 sept nanofils de silicium sont représentés, un focus étant effectué sur l'un de ces nanofils, d'une hauteur de 3,472 µm et d'un diamètre de 458 nm. La figure 3 présente le résultat d'une structuration directe sur un substrat massif de quartz, avant mise en œuvre des étapes de traitement de surfaces, d'isolation et de structuration. Sur cette figure 3 quatre nanofils de quartz (transparent) sont représentés. Ils présentent une hauteur de 5,90 µm environ et un diamètre de l'ordre de 2µm. La figure 4 expose un nanofil de Si d'une hauteur d'environ 13 µm, bien supérieure à la hauteur traditionnelle atteignable avec les procédés antérieurs. La figure 5 illustre une portion d'une plateforme comprenant sept nanofils et une ligne d'accès à l'issue de la mise en œuvre du procédé précédemment décrit. L'échelle de 5µm présentée sur la figure 5 permet de jauger de l'épaisseur constante de la ligne d'accès. La figure 6 est une vue de coupe de deux nanofils d'une hauteur identique à l'issue de la mise en œuvre du procédé précédemment décrit. La Figure 7 représente sept nanofils gravés dans une couche diélectrique de passivation de puce CMOS, un focus étant effectué sur l'un de ces nanofils, d'une hauteur de 2,22 µm et d'un diamètre de 520 nm. Les nanofils sont couverts d'une couche de Si amorphe déposé par PECVD à basse température (200 C).

La figure 8 représente une électrode à 3 nanofils où un dépôt localisé par électrochimie d'un matériau organique conducteur (PEDOT:PSS) a été réalisé.

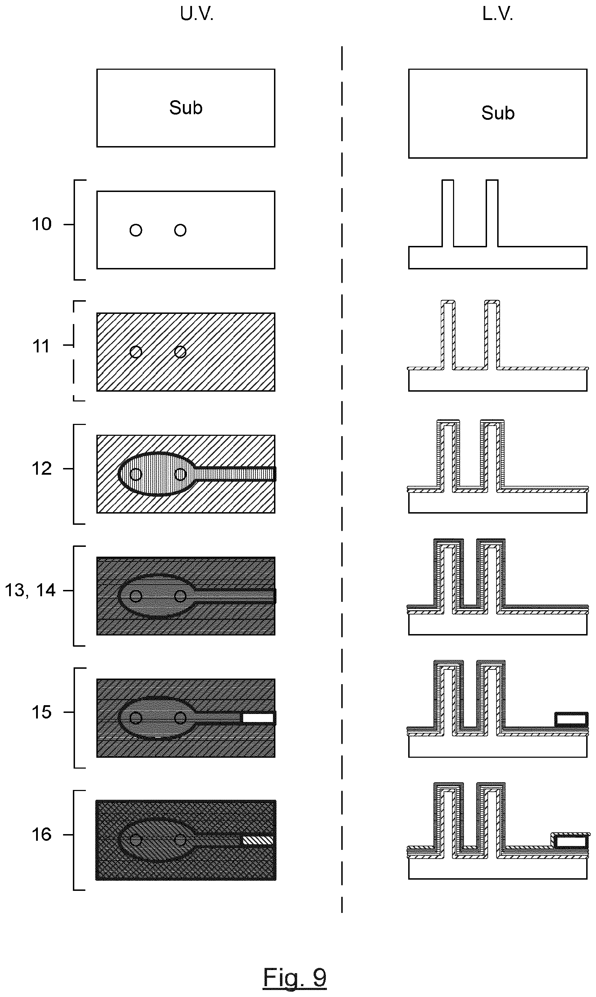

On présente en relation avec la figure 9, les étapes détaillées de fabrication d'une plateforme selon un mode de réalisation du procédé de fabrication présenté précédemment. En partie gauche, une vue supérieure (U.V.) du substrat et en partie droite une vue latérale (L.V.) du substrat sont représentées.

Plus particulièrement, en relation avec la figure 9, le procédé de fabrication comprend les étapes suivantes, qui sont réalisées sur une base comprenant un substrat (Sub) de silicium (Si) :
- fabrication (10) de nanofils verticaux : structuration verticale de la plateforme ;
   ∘ cette fabrication se base sur une technique de photolithographie, comprenant un dépôt local d'un motif de résine qui sert de masque de protection (nanoplot résistant de 500 nm de diamètre), en utilisant une résine organique conventionnelle (par exemple de type ex : ECI 3012) ; et
   ∘ gravure plasma (ICP-RIE gravure ionique réactive profonde) à la hauteur souhaitée (de 1 à 20 µm) : gravure sèche par bombardement d'ions (issus d'un plasma) ; cette technique présente l'avantage d'une forte anisotropie de la gravure: la frontière entre les zones gravées et non gravées est la plupart du temps rectiligne et verticale ; la résine restante est retirée par gravure chimique (résine issue de la sous étape précédente) ;
   ∘ à l'issue de cette première étape, on dispose sur la plateforme de nano fils verticaux, formant des groupes constitués de 1 à 100 nanofils et répartis sur la plateforme, qui sont retravaillés dans les étapes suivantes afin de leur adjoindre les propriétés attendues ;
- optionnellement, une couche d'isolant (oxydation thermique (11) sur Si ou dépôt d'oxyde) de l'intégralité du substrat nano structuré pour obtenir une couche isolante d'une épaisseur d'environ 100 nm (Cette étape n'est nécessaire que sur silicium ou autre substrat conducteur afin de pouvoir isoler les électrodes entre elles) ;
- création (12) des lignes d'accès :
   ∘ Dépôt silicium pleine plaque par CVD ; En effet, LPCVD : permet d'obtenir du Si poly mais à une température entre 500C° et 600C° - température qui n'est pas compatible avec une intégration sur CMOS, ou sur substrats flexible. Quant au PECVD, si amorphe, déposé entre 200C° et 300C°. Le Si est un peu moins qualitatif que par LPCVD (présence d'hydrogène dans la couche) mais les inventeurs ont pointé que cela est suffisant pour pouvoir réaliser l'alliage de Pt ou Ni (l'approche siliciuration sélective décrite par la suite) ;
   ∘ photolithographie, comprenant un dépôt local d'un motif de résine qui sert de masque de protection (motifs micrométrique définissant les contacts électriques connectant les nanosondes), en utilisant une résine organique conventionnelle dont l'épaisseur est supérieure à la longueur des nanostructures (par exemple de type ex : AZ4562) ; et
   ∘ gravure RIE : gravure sèche par bombardement d'ions (issus d'un plasma) ; cette technique présente l'avantage d'une forte anisotropie de la gravure: la frontière entre les zones gravées et non gravées sera la plupart du temps rectiligne et verticale ; l'intégralité des nanostructures étant protégées par la résine, la résine restante est retirée par gravure chimique (résine issue de la sous étape précédente) la gaine de Si sur les nanostructures n'est pas attaquée ;
   ∘ à l'issue de cette première étape, on dispose de groupe de nanostructures sur un substrat massif de Si couvert d'une couche isolante et d'une couche de Si. Sur les nanostructures on a une gaine de Si puis une gaine de SiO2 et enfin le cœur en Si. Le substrat massif de Si est couvert d'un isolant en SiO2 sur lequel des lignes d'accès sont structurées dans la couche de Si déposée permettant d'adresser les différents groupes de nanostructures indépendamment ;
- Création sélective d'un alliage Si-métal : exemple de la siliciuration (13) du platine (il est également possible de réaliser la même approche classiquement avec le Ni -et d'autres métaux Ti, etc. - le Ni permet de réaliser la réaction de siliciuration à plus basse température (intéressant pour les substrats plus sensibles à la température)) :
   ∘ pour créer des zones faiblement résistives et améliorer l'interface entre la nanosonde et le milieu liquide, une étape de dépôt isotrope de platine (Pt) pleine plaque est effectuée par PVD (de l'anglais pour « Physical Vapor Deposition ») ;
   ∘ un recuit d'activation (400°C, pendant 4 minutes) permet ensuite de créer l'alliage PtSi ; L'avantage ici est que le PtSi ne se crée que lorsque le Pt est en contact direct avec le Si et non du SiO2 : la technique employée permet donc de ne pas avoir de PtSi ailleurs qu'aux endroits désirés permettant ainsi de conserver la sensibilité de mesure de ceux-ci ; il s'agit d'un un processus de diffusion donc exponentiel avec la température. Il faut un certain temps à un temp donnée pour convertir une couche d'épaisseur e. Si la température est plus élevée, cela sera plus rapide. SI la couche est plus épaisse cela prendra plus de temps. 400C / 4 min permet de convertir une couche de 60 nm de Pt, ce qui convient à l'usage recherché.

Par conséquent, il n'est pas nécessaire de réaliser une étape de lithogravure pour l'implantation sur le métal : on n'a pas besoin de couvrir certaines zones de résine. De plus cette étape de siliciuration du platine permet d'augmenter fortement l'intégration des nanosondes, tout en autorisant une forte biocompatibilité avec le milieu vivant et ce grâce au recuit d'activation du platine sur le Si. Cette étape peut être réalisée avec d'autres métaux comme le Ni (qui présente une température de formation d'alliage inferieure), le Ti ...
- gravure (14) sélective du Pt par rapport au PtSi à l'eau régale (mélange chimique de HCl : HNO3 : EDI qui permet de graver seulement le métal : le siliciure n'est pas attaqué) :
   ∘ il s'agit d'une étape de gravure chimique sélective qui permet de graver le Pt non transformé (issu de l'étape précédente) sans attaquer le PtSi : le Pt (non transformé) est donc ôté du SiO2 ;
   ∘ L'intérêt du PtSi est double : du point de vue des nanosondes il ne s'oxyde pas (en comparaison du Si seul) et permet de conserver une faible impédance d'interface électrolyte/sonde dans le temps.
- métallisation (15) à l'aluminium (AI) permettant réduire la résistance des lignes d'accès :
   ∘ un dépôt conforme sur l'ensemble de la plateforme d'AI (500 nm) suivi d'une photolithographie et d'une gravure chimique de l'Al non protégé par la résine « etch-back » sont ensuite mis en œuvre pour métalliser les lignes d'accès ;
   ∘ l'Al n'est conservé que sur les lignes d'accès. La résine restante est retirée chimiquement ;
- isolation (16) de la plateforme par rapport au milieu :
   ∘ Un oxyde (oxyde d'isolation) est ensuite déposé de manière conforme pour isoler les nanofils de la plateforme : l'oxyde employé peut être du SiO2, de Al2O3 ou du HfO2, ou un diélectrique spécifique ;
   ∘ Un retrait sélectif de cet oxyde d'isolation est ensuite effectué sur les nanofils à proprement parlé ;
- finalement, une couche conductrice additionnelle peut être déposée sélectivement sur la nanostructure par dépôt électrochimique comme une couche organique conductrice (PEDOT:PSS) ou d'oxyde métallique (IrOx, RuOx) afin de pouvoir modifier les propriétés d'interface des nanosondes.

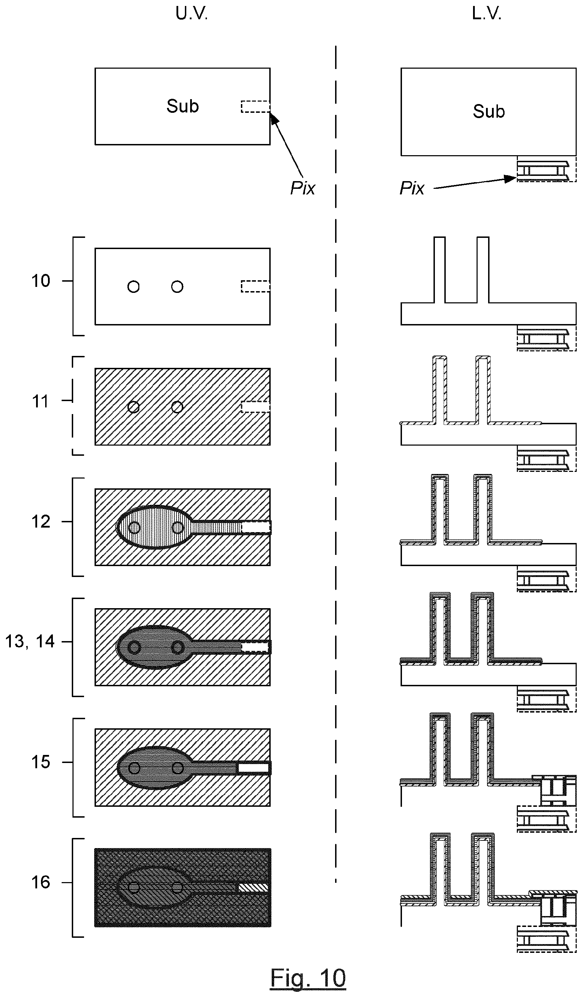

Dans une variante d'application de cet exemple de réalisation, décrit en relation avec la figure 10, le procédé précédemment décrit est mis en œuvre sur une puce de type circuit CMOS. Les étapes sont sensiblement identiques à celles décrites en relation avec la figure 9. Plus particulièrement, dans cette configuration, la couche de passivation diélectrique de la puce CMOS est utilisée tel un substrat massif, permettant de construire les nanofils (photolithographie, gravure plasma) directement sur cette couche de passivation diélectrique puis de créer les ligne d'accès, la siliciuration (etc.) sans avoir à adresser les pixels métalliques.

Sur la figure 10, des pixels du CMOS sont représentés (Pix), en vue schématique de coupe. Selon cette variante, les nanosondes sont construites au-dessus de pixels sélectionnés puis connectées lors de l'étape 15 de métallisation Al des lignes d'accès par un via (ouverture traversante) métallisé connectant ainsi chaque pixel sélectionné de la puce CMOS à sa nanosonde associée. Plus particulièrement, dans ce mode de réalisation, un schéma (pattern) spécifique est utilisé pour faire en sorte que les lignes d'accès métallisées des nanosondes entrent directement en contact avec les pixels de la puce. Ce schéma est par exemple fourni par le fabricant de la puce (lorsque celle-ci est préfabriquée en amont). Le schéma est ensuite utilisé lors de l'étape de structuration verticale de la plateforme pour non seulement créer les nanofils verticaux, comme explicité précédemment, mais également pour pratiquer une ouverture (via) complète de la couche de passivation, selon le schéma fourni, au-dessus de pixels avec lesquels les lignes d'accès doivent être connectées. Une fois cette reprise de contact par métallisation effectuée, l'isolation (16) de la plateforme par rapport au milieu est réalisée, ce qui permet d'isoler à nouveau la couche de passivation structurée du CMOS et de restituer les propriétés d'origine du CMOS. Un retrait sélectif de la couche d'isolation est ensuite effectué sur les nanosondes. Il est ainsi possible de créer une plateforme de nanosondes directement sur un composant électronique en charge d'effectuer tout ou partie des traitements des signaux électriques transitant par ces nanosondes.

Ainsi, on utilise directement la technologie CMOS pour développer une plateforme qui peut effectuer un couplage neuronal cellulaire et parallèle à grande échelle. Par exemple, le nombre de sites d'enregistrement de nanofils peut être significativement élevé, et ce réseau de nanofils est rendu « actif » en le fabriquant au-dessus du circuit intégré CMOS. Les nanofils dans chaque site d'enregistrement sont connectés à leur propre amplificateur et stimulateur dans le circuit intégré sous-jacent (c'est-à-dire que le circuit intégré lui-même assume une structure de réseau avec un nombre d'amplificateurs et/ou de stimulateurs équivalent au nombre de nanofils de la plateforme. L'électronique sur puce dans le circuit intégré peut être utilisée pour poursuivre plusieurs objectifs. En premier lieu, il rend possible le fonctionnement en parallèle du réseau de sites de nanofils à grande échelle. En deuxième lieu, l'électronique sur puce à proximité des nanofils permet d'augmenter la sensibilité d'enregistrement, par exemple en évitant un trajet trop long des signaux vers l'électronique, un tel trajet pouvant atténuer le signal ou introduire des bruits. Ainsi, une telle plateforme sur CMOS peut être utilisée pour l'enregistrement intracellulaire et massivement parallèle et la stimulation de cultures dissociées in vitro de réseaux de cellules. Une telle plateforme peut également être utilisé dans l'étude des réseaux neuronaux in vivo et le développement de nouveaux types de neuroprothèses. Comme exposé précédemment, l'avantage de la plateforme proposée, notamment obtenue avec le procédé de fabrication présenté, réside dans la très faible variabilité de fabrication, permettant réellement un passage à l'échelle et une réduction des coûts de fabrication.

De manière complémentaire, l'invention porte également sur une plateforme pour l'interfaçage cellulaire, fabriquée sur un substrat massif prédéterminé à partir de la méthode précédemment présentée. Notamment, une telle plateforme est remarquable en ce que la couche de silicium déposée postérieurement à la création des nanofils verticaux directement sur le substrat a une épaisseur comprise entre 20 et 200 nm ; avec une épaisseur préférée située aux alentours de 100 nm. Une telle plateforme peut également, selon les conditions de réalisation, comprendre des regroupements de nanofils verticaux en clusters, de sorte à produire des groupes prédéfinis pour la mesure cellulaire sur une même plateforme. La plateforme peut avantageusement être spécialisée en fonction des matériels biologiques à étudier/stimuler. Plus particulièrement, le positionnement et l'écartement des nanofils verticaux est déterminé et sélectionné en fonction des types de cellules à interfacer, et notamment de la taille de ces cellules. De plus, la surface de la plateforme peut être traitée de sorte à définir des zones hydrophiles/hydrophobes permettant un placement plus précis des cellules étudiées et permettant donc un meilleur interfaçage de ces cellules. Par ailleurs, la hauteur des nanofils sur ces plateformes est comprise entre 2 et 20 µm. La hauteur de ces nanofils est tout comme leur nombre et leur espacement, déterminée en fonction des typologies de cellules à interfacer., mais également du milieu dans lesquels ces cellules sont immergées afin de garantir leur vie/survie durant la phase d'étude. Par ailleurs, selon la présente, la hauteur des nanofils peut être différente sur une même plateforme, et ce afin de permettre à plusieurs typologies différentes de cellules de pouvoir être intégrées au sein d'une même cultures et/ou de pouvoir interfacer plusieurs couches de cellules d'un même échantillon biologique, assurant ainsi des mesures dans des conditions opérationnelles différentes, tout en permettant de conserver, dans une certaine mesure, une résolution de type cellule unique.

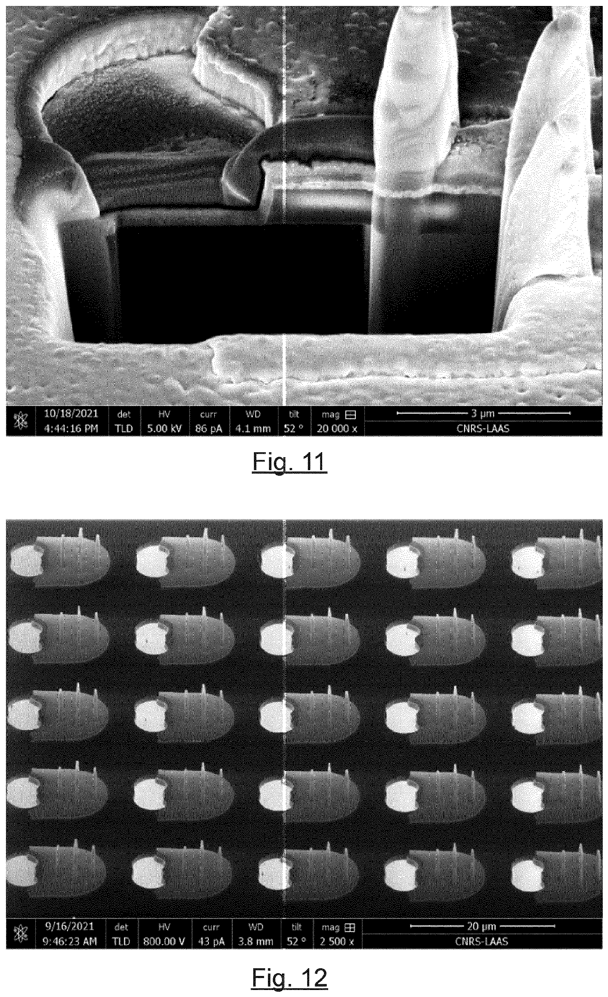

La figure 11 présente une vue de coupe d'un nanofil et de la via qui ont été fabriqués sur la couche de passivation du HDCMOS selon la technique précédemment proposée. La figure 12 représente un ensemble de sondes sur puce HDCMOS qui ont également été fabriqués par les inventeurs.

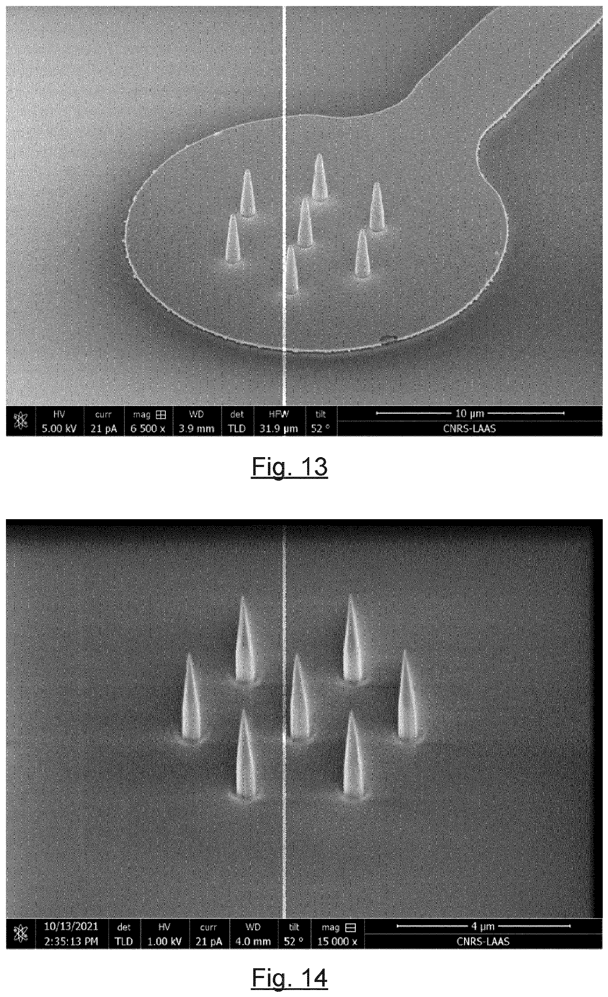

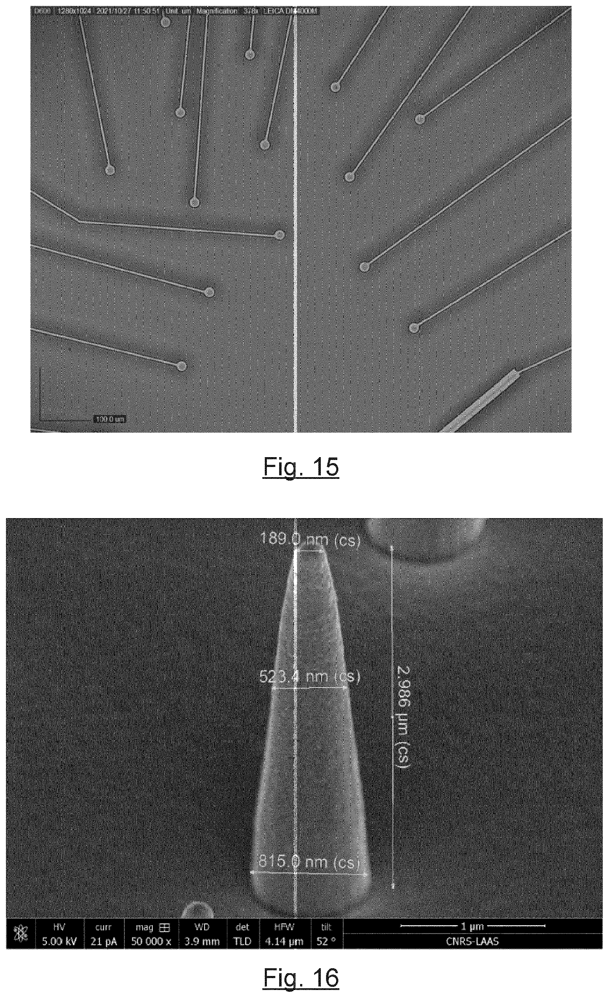

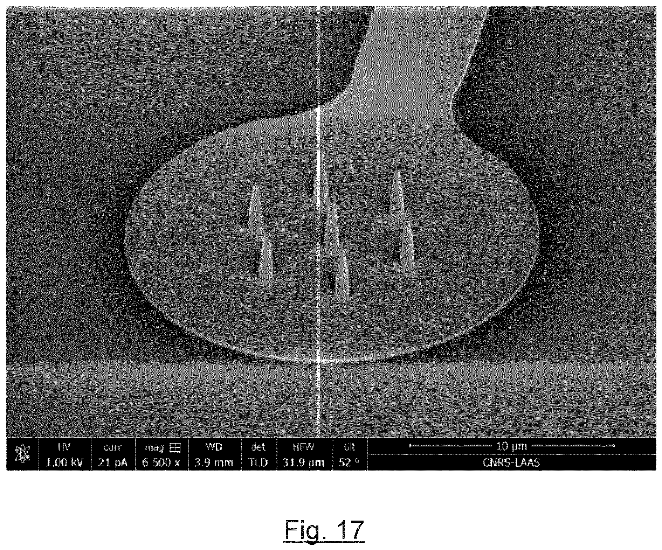

La figure 13 est une vue d'un ensemble de nanofils fabriqués sur une couche de quartz. La figure 14 est une vue d'un ensemble de nanofils fabriqués sur une couche de quartz avec une couche de l'alliage PtSi. La figure 15 est une vue d'un ensemble d'ensemble de nanofils fabriqués sur une couche de quartz avec une couche de l'alliage PtSi. La figure 16 est une vue rapprochée d'un nanofils fabriqués sur une couche de quartz avec une couche de l'alliage PtSi, précisant les dimensions de ce nanofil. La figure 17 est une vue d'un ensemble de nanofils fabriqués sur une couche de quartz avec une couche de Si.

Ainsi, comme il est prégnant à la vue des éléments présentés ci-dessus, les inventrices sont en possession d'une maitrise de la technologie décrite, permettant notamment d'envisager de nombreuses variations tant en termes de taille que de densité des nanofils, notamment construits sur substrat HDCMOS. Par ailleurs, à ce jour, aucune puce HDCMOS embarquant des plateformes d'interfaçage cellulaire sur leurs substrats n'a été divulguée dans l'art antérieur, les inventeurs pensent être seuls à disposer d'un tel microprocesseur comprenant une plateforme pour l'interfaçage cellulaire, cette plateforme comprenant au moins une nano-sonde à base de nanofils comprenant chacun une extrémité conductrice destinée à entrer en contact avec une cellule, processeur dans lequel les nanofils de la nanosonde sont directement positionnés et construits sur la couche de passivation du microprocesseur.

## Revendications

1. Procédé de fabrication d'une plateforme pour l'interfaçage cellulaire, ladite plateforme comprenant au moins une nanosonde et ladite plateforme étant fabriquée sur un substrat massif prédéterminé, ledit procédé étant de type descendant, **caractérisé en ce qu'**il comprend les étapes ordonnées suivantes :
- création (E10) de nanofils verticaux sur le substrat massif, destinés à former ladite au moins une nanosonde ;
- dépôt (E30) d'une couche de Si ;
- création (E40) des lignes d'accès aux nanofils ;
- siliciuration (E50) sélective des lignes d'accès et des nanofils ;
- structuration (E60) métallique des lignes d'accès ;
- dépôt (E60) d'une couche isolante pour la mesure en milieu liquide ;
- retrait sélectif (E70) de la couche isolante sur les nanofils de ladite au moins une nanosonde.

2. Procédé de fabrication d'une plateforme pour l'interfaçage cellulaire, selon la revendication 1, **caractérisé en ce que** le substrat massif prédéterminé appartient au groupe comprenant : substrat transparent, substrat flexible, silicium, une couche de passivation d'un circuit électronique CMOS.

3. Procédé de fabrication d'une plateforme pour l'interfaçage cellulaire, selon la revendication 1, **caractérisé en ce que** l'étape de création des nanofils sur le substrat massif prédéterminé comprend :
- une étape de photolithographie, comprenant un dépôt local d'un motif de résine qui sert de masque de protection ; et
- une étape de gravure sèche par bombardement d'ions permettant l'obtention des nanofils verticaux sur le substrat, à la hauteur souhaitée.

4. Procédé de fabrication d'une plateforme pour l'interfaçage cellulaire, selon la revendication 1, **caractérisé en ce que** l'étape de siliciuration sélective des nanofils est à base de platine, ou de nickel, ou de titane, ou de chrome, optionnellement recouvert d'une couche d'oxyde métallique (IrOx, RuOx) ou de conducteur organique (PEDOT:PSS) ou de nitrure métallique (TiN, TaN).

5. Procédé de fabrication d'une plateforme pour l'interfaçage cellulaire, selon la revendication 1, **caractérisé en ce que** l'étape de création des lignes d'accès aux nanofils comprend :
- Une étape de dépôt d'une couche de silicium, pleine plaque, par CVD ;
- Une étape de photolithographie, comprenant un dépôt local d'un motif de résine qui sert de masque de protection sur les lignes d'accès et les nanofils verticaux ; et
- gravure sèche par bombardement d'ions pour retrait de la couche de silicium aux emplacements non désirés.

6. Procédé selon la revendication 4, **caractérisé en ce que** la couche de silicium a une épaisseur comprise entre 20 et 200 nm.

7. Procédé selon la revendication 4, **caractérisé en ce que** la couche de silicium a une épaisseur d'environ 100 nm.

8. Procédé selon la revendication 1, **caractérisé en ce que** la hauteur des nanofils est comprises entre 2 et 20 µm

9. Plateforme pour l'interfaçage cellulaire, comprenant au moins une nano-sonde à base de nanofils verticaux comprenant chacun une extrémité conductrice destinée à entrer en contact avec une cellule, plateforme **caractérisée en ce qu'**elle comprend :
- un substrat massif transparent sur lequel, ou une couche de passivation d'un circuit électronique CMOS sur laquelle les nanofils sont directement structurés ;
- une couche active conductrice sur ledit substrat massif structuré ;
- des lignes d'accès auxdits nanofils ;
- une couche isolante recouvrant partiellement la plateforme, ladite couche isolante étant retirée au niveau des nanofils d'une ou plusieurs nanosondes,
**et en ce qu'**elle comprend une couche de siliciure sur ladite couche active, au niveau desdites lignes d'accès et desdits nanofils.

10. Plateforme pour l'interfaçage cellulaire selon la revendication 9, **caractérisée en ce qu'**elle comprend en outre une couche métallique sur ladite couche de siliciure desdites lignes d'accès, ladite couche métallique étant une couche d'oxyde métallique (IrOx, RuOx), un matériau conducteur organique (PEDOT:PSS), ou un métal nitruré (TiN, TaN).

11. Microprocesseur comprenant une plateforme selon la revendication 9 ou 10.

## Patentansprüche

1. Verfahren zur Herstellung einer Plattform zum Zell-Interfacing, wobei die Plattform mindestens eine Nanosonde umfasst und die Plattform auf einem vorgegebenen Grundsubstrat hergestellt wird, wobei das Verfahren vom Top-Down-Typ ist, **dadurch gekennzeichnet, dass** es die folgenden Schritte in der angegebenen Reihenfolge umfasst:
- das Erzeugen (E10) von senkrechten Nanodrähten auf dem Grundsubstrat, die zur Bildung der mindestens einen Nanosonde vorgesehen sind;
- das Abscheiden (E30) einer Si-Schicht;
- das Erzeugen (E40) von Anschlussleitungen zu den Nanodrähten;
- die selektive Silizidierung (E50) der Anschlussleitungen und der Nanodrähte;
- das metallische Strukturieren (E60) der Anschlussleitungen;
- das Abscheiden (E60) einer Isolierschicht zur Messung in einem flüssigen Medium;
- das selektive Entfernen (E70) der Isolierschicht auf den Nanodrähten der mindestens einen Nanosonde.

2. Verfahren zur Herstellung einer Plattform zum Zell-Interfacing nach Anspruch 1, **dadurch gekennzeichnet, dass** das vorgegebene Grundsubstrat zu der Gruppe gehört, die ein transparentes Substrat, ein biegsames Substrat, Silicium, eine Passivierungsschicht einer elektronischen CMOS-Schaltung umfasst.

3. Verfahren zur Herstellung einer Plattform zum Zell-Interfacing nach Anspruch 1, **dadurch gekennzeichnet, dass** der Schritt des Erzeugens von Nanodrähten auf dem vorgegebenen Grundsubstrat Folgendes umfasst:
- einen Fotolithografieschritt, der eine lokale Abscheidung einer Resiststruktur umfasst, die als Schutzmaske dient; und
- einen Trockenätzschritt durch Ionenbeschuss, der den Erhalt von senkrechten Nanodrähten auf dem Substrat mit der gewünschten Höhe ermöglicht.

4. Verfahren zur Herstellung einer Plattform zum Zell-Interfacing nach Anspruch 1, **dadurch gekennzeichnet, dass** der Schritt zur selektiven Silizidierung der Nanodrähte auf der Basis von Platin oder Nickel oder Titan oder Chrom erfolgt, das gegebenenfalls mit einer Schicht eines Metalloxids (IrOx, RuOx) oder eines organischen Leiters (PEDOT:PSS) oder eines Metallnitrids (TiN, TaN) überzogen wird.

5. Verfahren zur Herstellung einer Plattform zum Zell-Interfacing nach Anspruch 1, **dadurch gekennzeichnet, dass** der Schritt des Erzeugens von Anschlussleitungen auf den Nanodrähten Folgendes umfasst:
- einen Schritt des Abscheidens einer Voll-Wafer-Siliciumschicht mittels CVD;
- einen Fotolithografieschritt, der ein lokales Abscheiden einer als Schutzmaske dienenden Resiststruktur auf den Anschlussleitungen und den senkrechten Nanodrähten umfasst, und
- ein Trockenätzen durch Ionenbeschuss zum Entfernen der Siliciumschicht an den unerwünschten Stellen.

6. Verfahren nach Anspruch 4, **dadurch gekennzeichnet, dass** die Siliciumschicht eine Dicke zwischen 20 und 200 nm aufweist.

7. Verfahren nach Anspruch 4, **dadurch gekennzeichnet, dass** die Siliciumschicht eine Dicke von etwa 100 nm aufweist.

8. Verfahren nach Anspruch 1, **dadurch gekennzeichnet, dass** die Höhe der Nanodrähte zwischen 2 und 20 µm beträgt.

9. Plattform zum Zell-Interfacing, die mindestens eine Nanosonde auf der Basis von senkrechten Nanodrähten umfasst, die jeweils ein leitfähiges Ende umfassen, das dazu vorgesehen ist, in Kontakt mit einer Zelle zu gelangen, wobei die Plattform **dadurch gekennzeichnet ist, dass** sie Folgendes umfasst:
- ein transparentes Grundsubstrat oder eine Passivierungsschicht einer elektronischen CMOS-Schaltung, auf dem bzw. auf der die Nanodrähte direkt strukturiert sind;
- eine leitfähige aktive Schicht auf dem strukturierten Grundsubstrat;
- Anschussleitungen zu den Nanodrähten;
- eine Isolierschicht, welche die Plattform teilweise bedeckt, wobei die Isolierschicht im Bereich der Nanodrähte einer oder mehrerer Nanosonden entfernt ist,
und dadurch, dass sie eine Silicidschicht auf der aktiven Schicht im Bereich der Anschlussleitungen und der Nanodrähte umfasst.

10. Plattform zum Zell-Interfacing nach Anspruch 9, **dadurch gekennzeichnet, dass** sie außerdem eine Metallschicht auf der Silicidschicht der Anschlussleitungen umfasst, wobei es sich bei der Metallschicht um eine Schicht eines Metalloxids (IrOx, RuOx), eines organischen Leiters (PEDOT:PSS) oder eines Metallnitrids (TiN, TaN) handelt.

11. Mikroprozessor, der eine Plattform nach Anspruch 9 oder 10 umfasst.

## Claims

1. Method for manufacturing a platform for cellular interfacing, said platform comprising a least one nanoprobe and said platform being manufactured over a predetermined bulk substrate, said method being a top-down method, **characterised in that** it comprises the following steps in order:
- creating (E10) vertical nanowires over the bulk substrate;
- depositing (E30) a Si layer
- creating (E40) access lines for accessing the nanowires;
- selective silicidation (E50) of the access lines and of the nanowires;
- metal structuring (E60) of the access lines;
- depositing (E60) an insulating layer for liquid measurement;
- selective removal (E70) of the insulating layer on the nanowires of said at least one nanoprobes.

2. Method for manufacturing a platform for cellular interfacing, according to claim 1, **characterised in that** the predetermined bulk substrate belongs to the group comprising: transparent substrate, flexible substrate, silicon, a passivation layer of a CMOS electronic circuit.

3. Method for manufacturing a platform for cellular interfacing, according to claim 1, **characterised in that** the step of creating the nanowires over the predetermined bulk substrate comprises:
- a photolithography step, comprising a local deposition of a resin pattern which serves as a protective mask; and
- a step of dry etching by ion bombardment allowing obtaining vertical nanowires on the substrate, at the desired height.

4. Method for manufacturing a platform for cellular interfacing, according to claim 1, **characterised in that** the step of selective silicidation of the nanowires is based on platinum, or nickel, or titanium, or chromium, optionally covered with a layer of a metal oxide (IrOx, RuOx) or an organic conductor (PEDOT:PSS) or a metal nitride (TiN, TaN).

5. Method for manufacturing a platform for cellular interfacing, according to claim 1, **characterised in that** the step of creating the access lines to the nanowires comprises:
- A step of depositing a full-wafer silicon layer, by CVD;
- A photolithography step, comprising a local deposition of a resin pattern which serves as a protective mask over the access lines and the vertical nanowires; and
- dry etching by ion bombardment to remove the silicon layer at the undesirable locations.

6. Method according to claim 4, **characterised in that** the silicon layer has a thickness comprised between 20 and 200 nm.

7. Method according to claim 4, **characterised in that** the silicon layer has a thickness of about 100 nm.

8. Method according to claim 1, **characterised in that** the height of the nanowires is comprised between 2 and 20 µm.

9. Platform for cellular interfacing, comprising at least one nanoprobe based on nanowires each comprising a conductive end intended to come into contact with a cell, said platform being **characterised in that** it comprises:
- a transparent bulk substrate on which, or a passivation layer of a CMOS electronic circuit on which, the nanowires are directly structured;
- a conductive active layer on said structured bulk substrate;
- access lines to said nanowires;
- an insulating layer partially covering the platform, said insulating layer being removed at the level of the nanowires of one or more nanosensors,
and **in that** it comprises a silicide layer on said active layer, at the level of said access lines and said nanowires.

10. Platform for cellular interfacing according to claim 9, **characterised in that** it further comprises a metallic layer on said silicide layer of said access lines, said metallic layer being a metal oxide layer (IrOx, RuOx), an organic conductive material (PEDOT:PSS), or a nitrided metal (TiN, TaN)..

11. Microprocessor comprising a platform according to claim 9 or 10.
